(19) Europäisches Patentamt
European Patent Office
Office européen des brevets

(11) Veröffentlichungsnummer: **0 013 923**
**B1**

(12) # EUROPÄISCHE PATENTSCHRIFT

(45) Veröffentlichungstag der Patentschrift: **28.07.82**

(51) Int. Cl.³: **C 08 G 18/10,** C 08 G 18/72, C 08 G 18/78, C 07 C 127/19

(21) Anmeldenummer: **80100158.7**

(22) Anmeldetag: **14.01.80**

(54) **Suspensionen von Isocyanatoharnstoffen in Isocyanat-Präpolymeren, ein Verfahren zu ihrer Herstellung sowie ihre Verwendung bei der Herstellung von hochmolekularen Polyurethankunststoffen.**

(30) Priorität: **23.01.79 DE 2902469**

(43) Veröffentlichungstag der Anmeldung: **06.08.80 Patentblatt 80/16**

(45) Bekanntmachung des Hinweises auf die Patenterteilung: **28.07.82 Patentblatt 82/30**

(84) Benannte Vertragsstaaten: **BE DE FR GB IT NL SE**

(56) Entgegenhaltungen:

**DE-B-1 028 772**
**GB-A-842 338**
**GB-A-1 369 334**
**US-A-2 858 298**

(73) Patentinhaber: **BAYER AG, Zentralbereich Patente, Marken und Lizenzen, D-5090 Leverkusen 1, Bayerwerk (DE)**

(72) Erfinder: **Grögler, Gerhard, Dr., Von Diergardstrasse 48, D-5090 Leverkusen (DE)**
Erfinder: **Ganster, Otto, Dr., Berliner Strasse 64a, D-5090 Leverkusen (DE)**
Erfinder: **Recker, Klaus, Dr., Wolfskaul 6, D-5000 Koeln 80 (DE)**

**EP 0 013 923 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Suspensionen von Isocyanatoharnstoffen in Isocyanatpräpolymeren, ein Verfahren zu ihrer Herstellung sowie ihre Verwendung bei der Herstellung von hochmolekularen Polyurethankunststoffen

Die vorliegende Erfindung betrifft neuartige Suspensionen von Isocyanato-Harnstoffen in Isocyanatpräpolymeren, ein Verfahren zu ihrer Herstellung durch Umsetzung von Gemischen aus aromatischen Diisocyanaten mit Isocyanatgruppen unterschiedlicher Reaktivität und Präpolymeren mit endständigen Isocyanatgruppen mit Wasser, sowie die Verwendung der Suspensionen als Aufbaukomponente bei der Herstellung von Polyurethankunststoffen.

Es ist seit langem bekannt, dass die Reaktion von Wasser mit Monoisocyanaten zu substituierten Harnstoffen und mit Polyisocyanaten zu hochmolekularen Polyharnstoffen führt. Isocyanatgruppen enthaltende harzartige Polyharnstoffe kann man gemäss US-PS 2 597 025 dann erhalten, wenn in geeigneten Lösungsmitteln pro Mol aromatischem Diisocyanat 0,3-0,6 Mol $H_2O$ verwendet werden. Darüber hinaus ist bekannt geworden, dass aromatische Diisocyanate mit $H_2O$ auch selektiv zu niedermolekularen Diisocyanatoharnstoffen umgesetzt werden können. So erhält man gemäss US-PS 2 757 184, US-PS 2 757 185, US-PS 3 906 019 oder GB-PS 842 338 aus 2,4-Diisocyanatotoluol mit $H_2O$ unter geeigneten Reaktionsbedingungen den entsprechenden Bis-(3-isocyanatotolyl)-harnstoff.

Auch die analoge Reaktion von 2,6-Diisocyanatotoluol zu dem 1,3-Bis-(3-isocyanatotolyl)-harnstoff ist bekannt (US-PS 3 906 019, US-PS 2 902 474). Die DE-AS 1 028 772 oder die US-PS 2 858 298 beschreibt schliesslich die Verwendung derartiger Harnstoffdiisocyanate als Diisocyanatkomponente bei der Herstellung von hochmolekularen Polyurethankunststoffen, jedoch ohne jeglichen Hinweis auf die nachstehend näher beschriebenen erfindungsgemässen Suspensionen bzw. das Verfahren zu deren Herstellung.

Alle diese Verfahren zur Herstellung der Harnstoffdiisocyanate werden in Lösungsmittel durchgeführt. In diesen Lösungsmitteln müssen die Diiocyanate gut und das zugesetzte $H_2O$ zumindest teilweise löslich sein. Es darf keine polymerisierende Wirkung auf das Isocyanat ausüben und muss frei von gegenüber NCO-Gruppen reagierenden funktionellen Gruppen sein. Die Isocyanatharnstoffe fallen als schwer lösliche Verbindungen aus und werden durch Filtration isoliert. Für eine weitere Verarbeitung dieser Isocyanatharnstoffe für Polyurethan-Reaktionen ist es nötig, die durch Filtrieren gewonnenen und im Vakuum von dem Lösungsmittel befreiten Isocyanatharnstoffe durch entsprechende Mahlvorgänge in feinverteilte Form zu bringen. Infolge des hohen Schmelzpunktes und der Schwerlöslichkeit dieser Isocyanatharnstoffe werden sonst bei der weiteren Umsetzung oftmals inhomogene Produkte erhalten.

Isocyanatoharnstoffe, insbesondere niedermolekulare Bis-isocyanatoharnstoffe stellen besonders wertvolle Ausgangsmaterialien zur Herstellung von Polyurethankunststoffen dar, da die bei ihrer Verwendung eingebauten Harnstoffgruppierungen den Kunststoffen sehr gute mechanische Eigenschaften verleihen. Der vorliegenden Erfindung lag daher die Aufgabe zugrunde, Bis-isocyanatoharnstoffe in einem einfach durchzuführenden Verfahren in einer solchen Form zur Verfügung zu stellen, die eine einfache Weiterverarbeitung zu hochmolekularen Polyurethanen gestattet.

Überraschenderweise konnte diese Aufgabe dadurch gelöst werden, dass man aromatische Diisocyanate mit Isocyanatgruppen unterschiedlicher Reaktivität mit endständige Isocyanatgruppen aufweisenden Präpolymeren vermischt und anschliessend selektiv mit Wasser zum Bis-isocyanatoharnstoff umsetzt, der dann in Form einer leicht zu verarbeitenden Suspension in dem Präpolymeren anfällt. Es kann als überraschend angesehen werden, dass eine derartige selektive Reaktionsführung möglich ist, da Isocyanatgruppen aufweisende Voraddukte auch bei tiefer Temperatur bekanntlich mit Wasser vollständig unter Kohlendioxidentwicklung zu hochmolekularen Kunststoffen abreagieren. Es musste daher erwartet werden, dass eine selektive Überführung der aromatischen Diisocyanate in die entsprechenden Bis-isocyanatoharnstoffe in Gegenwart der Isocyanatgruppen aufweisenden Präpolymeren nicht möglich sein würde. Überraschenderweise findet jedoch bei der Durchführung des nachstehend näher beschriebenen erfindungsgemässen Verfahrens eine Kettenverlängerung des Präpolymeren praktisch nicht statt.

Gegenstand der vorliegenden Erfindung sind bei Raumtemperatur flüssige oder pastenförmige oder durch Erwärmen auf maximal 80°C verflüssigbare Suspensionen von

a) Isocyanatoharnstoffen der Formel

$$OCN\text{-}R\text{-}NH\text{-}CO\text{-}NH\text{-}[R\text{-}NH\text{-}CO\text{-}NH]_m\text{-}R\text{-}NCO$$

in welcher

R für einen zweiwertigen aromatischen Rest steht, wie er durch Entfernung der Isocyanatgruppen aus einem aromatischen Diisocyanat des Molekulargewichtsbereichs 174-400 mit einer sterisch ungehinderten aromatisch gebundenen Isocyanatgruppe und einer, durch mindestens einen Substituenten in o-Stellung sterisch gehinderten aromatisch gebundenen Isocyanatgruppe erhalten wird, wobei jedoch auch bis zu 50% der Reste R für einen solchen zweiwertigen aromatischen Rest stehen können, wie er durch Entfernung der Isocyanatgruppen aus einem aromatischen Diisocyanat mit Isocyanatgruppen gleicher Reaktivität erhalten wird und

m für 0 oder eine ganze Zahl von 1 bis 5 steht, in

b) Isocyanat-Präpolymeren der Formel

$$D-(O-CO-NH-A-NCO)_n$$

in welcher

A für einen gegebenenfalls Alkyl-substituierten aromatischen Kohlenwasserstoffrest mit insgesamt 6-15 Kohlenstoffatomen, einen aliphatischen Kohlenwasserstoffrest mit 4-10 Kohlenstoffatomen, einen cycloaliphatischen Kohlenwasserstoffrest mit 4-15 Kohlenstoffatomen oder einen Xylylenrest steht,

D für einen Ester- oder Äthergruppen aufweisenden Rest steht, wie er durch Entfernung der Hydroxylgruppen aus einem Polyester- oder Polyätherpolyol des Molekulargewichtsbereichs 500-8000 erhalten wird, und

n für eine ganze Zahl von 2 bis 4 steht.

Gegenstand der vorliegenden Erfindung ist auch ein Verfahren zur Herstellung dieser Suspensionen, welches dadurch gekennzeichnet ist, dass man 5-40 Gew.-%, bezogen auf Gesamtgemisch, an aromatischen Diisocyanaten der Formel

$$R(NCO)_2$$

mit Isocyanatpräpolymeren der Formel

$$D-(O-CO-NH-A-NCO)_n$$

vermischt und anschliessend das Gemisch mit 0,4 bis 0,8 Mol Wasser pro Mol des aromatischen Diisocyanats oder einer entsprechenden Menge einer wasserabspaltenden Verbindung bei einer Temperatur zwischen 20 und 80°C zur Reaktion bringt, wobei A, D, R und n die in Anspruch 1 genannte Bedeutung haben.

Gegenstand der vorliegenden Erfindung ist auch die Verwendung der erfindungsgemässen Suspensionen als Isocyanatkomponente bei der Herstellung von Polyurethankunststoffen nach dem Isocyanat-Polyadditionsverfahren.

Die in obengenannten Formeln aufgeführten Variablen haben auch nachstehend die obengenannte Bedeutung. Vorzugsweise stehen

A für einen Rest der bei der Definition von A bereits genannten Art,

D für einen Ester- oder Äthergruppen aufweisenden Rest, wie er durch Entfernung der Hydroxylgruppen aus einem Polyester- oder Polyätherdiol des Molekulargewichtsbereiches 500 bis 8 000, insbesondere 1 000 bis 3 000 erhalten wird,

R für einen 2,4-Tolyulrest,

m für 0 oder eine ganze Zahl von 1 bis 3 und

n für 2.

Unter aromatischen Diisocyanaten mit Isocyanatgruppen unterschiedlicher Reaktivität sind im Rahmen der vorliegenden Erfindung vor allem der strikten Bedeutung dieser Definition entsprechende Diisocyanate, jedoch auch Gemische derartiger Diisocyanate mit bis zu 50 Gew.-%, bezogen auf Gesamtgemisch an aromatischen Diisocyanaten mit Isocyanatgruppen gleicher Reaktivität zu verstehen.

Für das erfindungsgemässe Verfahren geeignete Diisocyanate

$$R(NCO)_2$$

sind demzufolge beispielsweise solche, welche neben einer freien, d.h. sterisch ungehinderten aromatisch gebundenen Isocyanatgruppe eine weitere aromatisch gebundene Isocyanatgruppe aufweisen, welche durch mindestens einen Substituenten in o-Stellung sterisch gehindert ist. Derartige Substituenten, die zu einer sterischen Hinderung der Isocyanatgruppe führen können, sind insbesondere $C_1$-$C_8$-Alkyl-, $C_6$-$C_{10}$-Aryl-, $C_1$-$C_8$-Alkoxy-, $C_1$-$C_8$-Alkoxycarbonyl-, Chlor-, Brom- oder Cyano-Gruppen. Desweiteren ist eine sterische Hinderung der automatisch gebundenen Isocyanatgruppen dann gegeben, wenn das Grundgerüst des Diisocyanats ein gegebenenfalls über Brückenglieder wie Alkylen-, Äther-, Sulfoxid- oder Sulfongruppen verknüpftes System mehrerer aromatischer Ringe darstellt und die (sterisch gehinderte) Isocyanatgruppe in ortho-Stellung zu dem 2 aromatische Ringe verknüpfenden Brückenglied angeordnet ist.

Besonders bevorzugte aromatische Diisocyanate sind beispielsweise solche der Formeln (I) und (II).

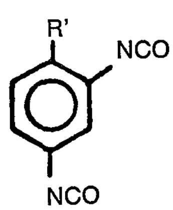

(I)

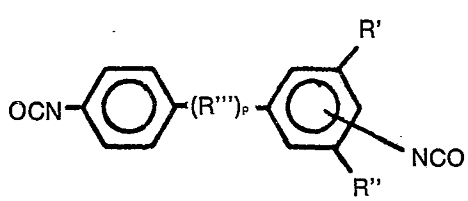

(II)

wobei

R' und R'' für die gleiche oder verschiedene Reste stehen und eine die sterische Hinderung der Isocyanatgruppe bewirkende Gruppe der oben beispielhaft genannten Art darstellen, wobei bei den Produkten der Formel (II) einer der Reste R' bzw. R'' auch für Wasserstoff stehen

R' und R'' für gleiche oder verschiedene kann, bzw. wobei in der Formel (II) auch beide R' und R'' für Wasserstoff stehen können,

falls die Isocyanatgruppe ortho-ständig zur Brücke R''' bzw. im Falle von p = 0 ortho-ständig zum linken aromatischen Rest angeordnet ist;

R''' für eine die aromatischen Ringe verknüpfende Brücke der oben beispielhaft genannten Art und

p für 0 oder 1 steht.

Es ist auch möglich, beim erfindungsgemässen Verfahren Gemische aromatischer Diisocyanate einzusetzen, in welchen aromatische Diisocyanate mit Isocyanatgruppen gleicher Reaktivität wie beispielsweise 4,4'-Diisocyanatodiphenylmethan oder 2,6-Diisocyanatotoluol vorliegen, falls der Anteil derartiger Diisocyanate mit Isocyanatgruppen gleicher Reaktivität eine obere Grenze von 50, vorzugsweise von 40 Gew.-% bezogen auf das Gesamtgemisch nicht übersteigt.

Zu den besonders bevorzugten, für das erfindungsgemässe Verfahren geeigneten Diisocyanaten gehören z.B. 2,4-Diisocyanatotoluol, gegebenenfalls im Gemisch mit bis zu 50 Gew.-% bezogen auf Gesamtgemisch an 2,6-Diisocyanatotoluol, 2,4'-Diisocyanatodiphenylmethan, gegebenenfalls im Gemisch mit bis zu 50 Gew.-% bezogen auf Gesamtgemisch an 4,4'-Diisocyanatodiphenylmethan. Weiterhin geeignet sind z.B. 2,4'-Diisocyanatodiphenylpropan, 2,4'-Diisocyanatodiphenyläther, 2,4'-Diisocyanatodiphenylsulfon, 2,4'-Diisocyanatodiphenylsulfodioxid, 3-Methyl-4,4'-diisocyanatodiphenylmethan, 3-Äthyl-4,4'-diisocyanatodiphenylmethan, 3-Isopropyl-4,4'-diisocyanatodiphenylmethan, 3,5-Dimethyl-4,4'-diisocyanatodiphenylmethan, 3,5-Diäthyl-4,4'-diisocyanatodiphenylmethan oder 3,5-Diisopropyl-4,4'-diisocyanatodiphenylmethan, 3-Carboxymethyl-4,4'-diisocyanatodiphenylmethan, 3-Carboxyäthyl-4,4'-diisocyanatodiphenylmethan.

Zur Herstellung der Isocyanat-Präpolymeren geeignete Polyole der Formel

$$D\ (OH)_n$$

sind 2 bis 4, vorzugsweise 2 Hydroxylgruppen aufweisende Polyester- bzw. Polyätherpolyole des Molekulargewichtsbereichs 500 bis 8 000, vorzugsweise 1 000 bis 3 000, wie sie für die Herstellung von homogenen oder geschäumten Polyurethanen an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein. Als Beispiele hierfür seien genannt: Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester und Terephthalsäure-bis-glykolester. Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, Cyclohexandimethanol (1,4-Bis-hydroxymethylcyclohexan), 2-Methyl-1,3-propandiol, Glycerin, Trimethylolpropan, Hexantriol-(1,2,6), Butantriol-(1,2,4), Trimethyloläthan, ferner Diäthylenglykol, Triäthylenglykol, Tetraäthylenglykol, Polyäthylenglykole, Dipropylenglycol, Polypropylenglykole, Dibutylenglykol und Polybutylenglykole in Frage. Auch Polyester aus Lactonen, z.B. ε-Caprolacton oder Hydroxycarbonsäuren, z.B. ω-Hydroxycapronsäure, sind einsetzbar.

Auch die erfindungsgemäss in Frage kommenden, zwei bis vier vorzugsweise zwei Hydroxylgruppen aufweisenden Polyäther sind solche der an sich bekannten Art und werden z.B. durch Polymerisation von Epoxiden wie Äthylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole oder Amine, z.B. Äthylenglykol, Propylenglykol-(1,3) oder -(1,2), Trimethylolpropan, 4,4'-Dihydroxydiphenylpropan, Anilin, Ammoniak, Äthanolamin oder Äthylendiamin hergestellt. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch durch Vinylpolymerisate modifizierte Polyäther, wie sie z.B. durch Polymerisation von Styrol und Acrylnitril in Gegenwart von Polyäthern entstehen (amerikanische Patentschriften 3 383 351, 3 304 273, 3 523 093, 3 110 695, deutsche Patentschrift 1 152 536), sind geeignet.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diäthylenglykol, Triäthylenglykol oder Tetraäthylenglykol mit Diarylcarbonaten, z.B. Diphenylcarbonat, oder mit Phosgen hergestellt werden können.

Vertreter dieser erfindungsgemäss zu verwendenden Verbindungen sind z.B. in High Polymers, Vol. XVI, «Polyurethanes, Chemistry and Technology», verfasst von Saunders-Frisch, Interscience Publishers, New York, London, Band

I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z.B. auf den Seiten 45 bis 71 beschrieben.

Selbstverständich können Mischungen der obengenannten Verbindungen, z.B. Mischungen von Polyäthern und Polyestern, eingesetzt werden.

Zur Herstellung der Isocyanat-Präpolymeren geeignete Diisocyanate der Formel

$$A\ (NCO)_2$$

sind beispielsweise Tetramethylendiisocyanat, Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan, 2,4- und 2,6-Hexahydrotoluylendiisocyanat sowie beliebige Gemische dieser Isomeren, Hexahydro-1,3-und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Carbodiimidgruppen aufweisende Polyisocyanate, wie sie in der deutschen Patentschrift 1 092 007 beschrieben werden, Diisocyanate, wie sie in der amerikanischen Patentschrift 3 492 330 beschrieben werden. Ferner ist es möglich, beliebige Mischungen dieser Diisocyanate zu verwenden.

Besonders bevorzugt werden in der Regel die technisch leicht zugänglichen Diisocyanate, z.B. das 2,4- und 2,6-Toluylendiisocyanat sowie beliebige Gemische dieser Isomeren («TDI»), der 2,4'- und/oder 4,4'-Diisocyanatodiphenylmethan eingesetzt.

Aus den beispielhaft genannten Polyhydroxylverbindungen $D(OH)_n$ und Diisocyanaten $A(NCO)_2$ werden in an sich bekannter Weise die als Ausgangsmaterial beim erfindungsgemässen Verfahren einzusetzenden Isocyanat-Präpolymeren der Formel

$$D\text{-}(O\text{-}CO\text{-}NH\text{-}A\text{-}NCO)_n$$

hergestellt. Hierbei werden im allgemeinen überschüssige Mengen der Diisocyanate mit den Polyolen zur Reaktion gebracht, worauf sich oftmals ein Abdestillieren des nicht umgesetzten Diisocyanat-Überschusses anschliesst. Die Molverhältnisse der Reaktionspartner werden dabei so gewählt, dass eine Kettenverlängerungsreaktion nicht oder nur in untergeordnetem Umfang eintritt, d.h. es wird vorzugsweise mindestens in einem molaren NCO/OH-Verhältnis von 2:1 gearbeitet.

Die beim erfindungsgemässen Verfahren einzusetzenden Präpolymeren sind entweder bei Raumtemperatur flüssig oder durch einfaches Erwärmen auf maximal 80°C verflüssigbar.

Zur Durchführung des erfindungsgemässen Verfahrens wird das aromatische Diisocyanat

$$R\ (NCO)_2$$

dem Präpolymeren der Formel

$$D\text{-}(O\text{-}CO\text{-}NH\text{-}A\text{-}NCO)_n$$

zugemischt, wobei die Menge des Diisocyanats so bemessen wird, dass, bezogen auf Gesamtgemisch, 5 bis 40, vorzugsweise 5 bis 25 Gew.-% des Diisocyanats im Gemisch vorliegen. Anschliesend wird dem so erhaltenen Gemisch Wasser oder eine wasserabspaltende Verbindung zugesetzt, wobei pro Mol an aromatischem Diisocyanat $R(NCO)_2$ 0,4 bis 0,8, vorzugsweise 0,5 bis 0,6 Mol Wasser zum Einsatz gelangen. Anstelle von Wasser können wasserabspaltende Verbindungen wie z.B. Ameisensäure, tert.-Alkohole wie z.B. tert.-Butanol, kristallwasserhaltige organische oder anorganische Verbindungen wie Pinakonhexahydrat, Chloralhydrat oder Natriumsulfat-decahydrat zum Einsatz gelangen.

Die erfindungsgemässe Umsetzung erfolgt im Temperaturbereich zwischen 20 und 80°C, vorzugsweise bei 40 bis 60°C.

Je nach Viskosität der Ausgangsmaterialien, sowie in Abhängigkeit von der Konzentration an sich bildendem Harnstoffdiisocyanat werden bei Raumtemperatur flüssige, pastenförmige oder durch Erwärmen auf maximal 80°C verflüssigbare Suspensionen der Harnstoffdiisocyanate der Formel

$$OCN\text{-}R\text{-}NH\text{-}CO\text{-}NH\text{-}[R\text{-}NH\text{-}CO\text{-}NH]_m\text{-}R\text{-}NCO$$

erhalten, wobei m in Abhängigkeit vom gewählten Isocyanat/Wasser-Molverhältnis einem Wert innerhalb des obengenannten Bereichs entspricht. Bei NCO-Voraddukten höherer Polarität und daher besserer Lösefähigkeit für den sich in situ bildenden Polyisocyanatharnstoff erfolgt die Ausbildung einer dispersen Phase zuweilen erst nach längerem Stehen oder durch Animpfung. Eine besondere Verfahrensweise besteht darin, dass man zur Darstellung des höhermolekularen NCO-Voradduktes gleich solche niedermolekularen Diisocyanate verwendet, die verschieden reaktive NCO-Gruppen besitzen. Bei der Herstellung dieser NCO-Voraddukte wird das NCO/OH Verhältnis so eingestellt, dass nach Beendigung der Polyol/Isocyanatreaktion noch ein entsprechender Überschuss an freiem aromatischem Diisocyanat vorliegt.

Die Reaktion des Wassers mit dem überschüssigen aromatischen Diisocyanat läuft im allgemeinen so vollständig ab, dass die entstehenden Suspensionen nur noch geringe Restmengen an freiem Diisocyanat enthalten. Dies ist insbesondere bei Verwendung von Diisocyanaten mit relativ hohem Dampfdruck (z.B. Diisocyanatotoluol) von physiologischer Bedeutung. Man erhält insbesondere bei einem mo-

larem $H_2O$/NCO-Verhältnis (bezogen auf aromatisches Diisocyanat) von 1/4 - 1/2,6 monomerenarme Suspensionen mit freiem Isocyanatgehalt von kleiner als 0,6 Gew.-%. Eine nennenswerte Vernetzung der höhermolekularen NCO-Voraddukte durch Wasser tritt dabei überraschenderweise nicht ein.

Grundsätzlich ist es auch möglich, beim erfindungsgemässen Verfahren über 80°C schmelzende NCO-Präpolymere einzusetzen, hierbei empfiehlt sich dann insbesondere die Verwendung von wasserabspaltenden Verbindungen wie z.B. von Pinakonhexahydrat anstelle von Wasser, sowie die Mitverwendung von desaktivierenden aciden Verbindungen wie z.B. Phosphorsäure, Toluolsulfonsäure oder Benzylchlorid in Mengen von 0,01 bis 0,1 Gew.-%, bezogen auf Reaktionsgemisch, zur Verhinderung von Nebenreaktionen (Biuretbildung). Auch bei Verwendung von tert. Butanol als Wasserabspalter sind diese Verbindungen erforderlich, um die Spaltungstemperatur des zunächst entstehenden tert.-Butylurethans herabzusetzen.

Die literaturbekannten, Diisocyanat-Additionsreaktion beschleunigenden Verbindungen wie tert. Amine oder metallorganische Verbindungen werden bei der Durchführung des erfindungsgemässen Verfahrens im allgemeinen nicht mitverwendet, um die Bildung von hochmolekularen, keine Isocyanatgruppen aufweisenden, Polyharnstoffen zu verhindern.

Der Reaktionsverlauf kann durch Bestimmung des Gasvolumens (Kohlendioxid-Entwicklung) leicht verfolgt und kontrolliert werden. Die Diisocyanatoharnstoffe fallen nach Überschreiten der Löslichkeitsgrenze in fein verteilter Form aus, und man erhält vorwiegend sedimentstabile Suspensionen. Der Gesamt-NCO-Anteil dieser Suspensionen setzt sich zusammen aus dem NCO-Gehalt des höhermolekularen Voradduktes und dem NCO-Anteil des suspendierten Diisocyanatharnstoffes, er liegt im allgemeinen bei 5 bis 15, vorzugsweise 5 bis 10 Gew.-%.

Bei der erfindungsgemässen Verwendung der erfindungsgemässen Suspensionen können die vorliegenden unterschiedlichen Isocyanatgruppen je nach Arbeitsbedingungen selektiv zur Reaktion gebracht werden. So erfolgt bei der Glykol-, Amin- oder Wasservernetzung zunächst ein rascher Aufbau einer hochviskosen oder festen PU-Matrix infolge Vernetzung des homogenen vorliegenden NCO-Voradduktes. Vielfach verzögert erfolgt anschliessend der Umsatz des in heterogener Phase vorliegenden Polyisocyanatharnstoffes mit dem Kettenverlängerer. Die Zeitfolge dieser beiden Vernetzungsschritte kann durch das Mass der Schwerlöslichkeit und den Schmelzpunkt des Polyisocyanatharnstoffes, durch die Polarität bzw. das Lösungsvermögen des NCO-Adduktes sowie durch die Vernetzungstemperatur gesteuert werden. Auf Wunsch kann dieser zweite Reaktionsschritt auch auf einen beliebigen Zeitpunkt verschoben werden. Die Endvernetzung des noch nicht ausreagierenden Systems kann dann gegebenenfalls bei erhöhter Temperatur unter Formgebung an einem beliebigen Ort durchgeführt werden.

Ein weiterer Vorteil der erfindungsgemässen Suspensionen besteht darin, dass man auch bei der Vernetzung mit einem Diol oder gegebenenfalls Triol Formkörper erhält, die bereits einen entsprechenden Anteil an stabilen Harnstoffgruppen enthalten, die bekanntlich zu einer Verbesserung der mechanischen Werte und des Wärmestandes der PU-Systeme beitragen. Hierbei wird durch den verzögerten zweiten Reaktionsschritt die Reaktivität und somit die Giesszeit (Topfzeit) der Ansätze günstig beeinflusst, da nicht der gesamte NCO-Anteil mit dem Polyol (bzw. Diamin) auf einmal abreagiert.

Weiterhin sind auch die erfindungsgemässen Suspensionen auf Basis von aromatischen Diisocyanaten mit relativ hohem Dampfdruck, wie z.B. Diisocyanatotoluol, infolge ihres geringen Monomerenanteiles als physiologisch unbedenklich zu betrachten. Je nach Menge des Polyisocyanatharnstoffes im NCO-Voraddukt erhält man nach der Vernetzung mit Glykolen, Wasser oder Aminen verschiedene Härteeinstellungen.

Zur Vernetzung der erfindungsgemässen Polyisocyanatoharnstoff-Suspensionen kommen als Kettenverlängerungsmittel neben Wasser insbesondere Glykole mit einem Molekulargewicht zwischen 62 und 500 in Frage. Als Beispiele für derartige Verbindungen seien genannt: Äthylenglykol, Propylenglykol-(1,2) und -(1,3), Butandiol-(1,4) und -(2,3), Pentandiol-(1,5), Hexandiol-(1,6), Octandiol-(1,8), Neopentylglykol, 1,4-Bishydroxymethyl-cyclohexan, 2-Methyl-1,3-propandiol, Butendiol, Butindiol, Monochlorhydrin, Glycerin-monoalkyl- oder -monoaryl-äther, Xylylenglykole, das Diels-Alder-Anlagerungsprodukt von Butendiol an Anthracen, Chinit, Hexahydrobrenzcatechin, 4,4'-Dihydroxydiphenylpropan, Di-hydroxymethyl-hydrochinon, Hydrochinon-bis-hydroxyäthyläther, Diäthylenglykol, Triäthylenglykol, Tetraäthylenglykol, höhere Polyäthylenglykole mit einem Molekulargewicht bis 500, Dipropylenglykol, höhere Polypropylenglykole mit einem Molekulargewicht bis 500, Dibutylenglykol, höhere Polybutylenglykole mit einem Molekulargewicht bis 500 und N-Methyldiäthanolamin. Besonders geeignet sind 1,4- und 2,3-Butandiol, cyclische Glykole wie Hexahydrobrenzcatechin und Hydrochinon-bis-hydroxyäthyläther und Thiodiglykol.

Bei der Verwendung von Wasser als Kettenverlängerungsmittel erhält man geschäumte zellige Formteile. Um die Bildung eines Schaumstoffes zu vermeiden, kann das geschäumte Produkt in an sich bekannter Weise unter Druck und Formgebung verpresst werden.

Für die Aminvernetzung seien als Beispiele für aromatische Diamine Bisanthranilsäureester gemäss den DE-OSen 2 040 644 und 2 160 590, 3,5- und 2,4-Diaminobenzoesäureester gemäss DE-OS 2 026 900, die in den DE-OSen 1 803 635, 2 040 650 und 2 160 589 beschriebenen estergruppenhaltigen Diamine, sowie 3,3'-Dichlor-4,4'-diamino-diphenylmethan, 3,3'-Dithioäther-4,4'--diaminodiphenylmethan, Phenylendiamine, Toluylendiamine, 3,5-Diäthyl-2,4-diaminotoluol, 4,4'-Diaminodiphenylmethan und 4,4'- oder 2,2'-Diaminodiphenyldisulfid genannt.

Gegebenenfalls können in jeder Stufe des erfindungsgemässen Verfahrens bzw. der erfindungsgemässen Verwendung auch an sich bekannte Weichmacher, Farb- und Füllstoffe zugegeben werden. Geeignete Weichmacher sind beispielsweise Phthalsäureester und organische Sulfonamide. Besonders günstig sind häufig Schwefel enthaltende Weichmacher wie z.B. Methylen-bis-thioglykolsäurebutylester. Wie bei Naturkautschuk bewirken manche Füllstoffe eine Verbesserung der mechanischen Eigenschaften der erfindungsgemäss hergestellten Polyurethan-Elastomeren. Dies trifft z.B. auf Titandioxd, Siliciumdioxid, Bentonit, Calciumsilicat und Russ zu. Diese Füllstoffe können z.B. direkt in die höhermolekulare Polyhydroxylverbindung oder auch in das NCO-Präpolymer eingearbeitet werden.

Die erfindungsgemäss hergestellten Polyurethan-Elastomeren weisen ein hervorragendes mechanisches Eigenschaftsbild und eine ausgezeichnete Beständigkeit gegenüber organischen Lösungsmitteln und Ölen auf. Diese Eigenschaften eröffnen den erfindungsgemäss hergestellten Elastomeren ein breites Einsatzgebiet, beispielsweise als Walzenbeläge, elastische Bauteile für Maschinen, Dichtungen, Puffer, Faltenbälge, Beläge für Kugelmühlen, Schuhsohlen, Zahnräder und Fahrzeugreifen.

Die nachfolgenden Beispiele erläutern die vorliegende Erfindung. Wenn nicht anders vermerkt, sind Mengenangaben als Gewichtsteile bzw. Gewichtsprozente zu verstehen.

Beispiel 1

Zu 2348 g (1 Mol) eines NCO-Präpolymeren, das aus 2000 g eines linearen Polypropylenglykoläthers vom Mol-Gewicht 2000 (OH-Zahl = 56) und 348 g (2,0 Mol) 2,4-Diisocyanatotoluol hergestellt wurde und einen NCO-Gehalt von 3,5% aufweist, werden 174 g (1,0 Mol) 2,4-Diisocyanatototolul zugemischt. Zu dieser Mischung werden bei einer Temperatur von 50-60°C innerhalb 1/2 Stunde 9 g (0,5 Mol) Wasser zugesetzt und der Reaktionsansaz nach 5-6 Stunden bei dieser Temperatur gehalten. Nach Beendigung der $CO_2$-Entwicklung (11 l) erhält man eine Polyisocyanatharnstoff-Suspension mit dem Gesamt-NCO-Gehalt von 4,9% und einer Viskosität von 10 500 mPa . s/25°C.

Beispiel 1b

Werden obigen NCO-Präpolymeren 348 g (2,0 Mol) 2,4-Diisocyanatotoluol zugesetzt und die Mischung bei 60°C mit 18 g (1,0 Mol) Wasser umgesetzt, so erhält man nach 8-10 Stunden eine Polyisocyanatharnstoff-Suspension mit einem Gesamt-NCO-Gehalt von 6,0% und einer Viskosität von 18 000 mPa . s/25°C. Es werden 23 l $CO_2$ entwickelt. Der aus dieser Suspension durch Ausfällen mit Äther isolierte Polyisocyanatharnstoff zeigt einen NCO-Gehalt von 16,5%.

Beispiel 2

Zu einer Mischung aus 1 000 g des im Beispiel 1 genannten NCO-Präpolymeren vom NCO-Gehalt 3,5% und

| | | |
|---|---|---|
| a) 174 g (1,0 Mol) | = | 2,4-Diisocyanatotoluol |
| b) 174 g (1,0 Mol) | = | Diisocyanatotoluol (2,4- und 2,6-Isomeres im Verhältnis 80 = 20) |
| c) 174 g (1,0 Mol) | = | Diisocyanatotoluol (2,4- und 2,6-Isomeres im Verhältnis 65 = 35) |

werden jeweils 9 g (0,5 Mol) Wasser zugesetzt und die Reaktionsansätze bis zur Beendigung der $CO_2$-Entwicklung bei 50-60° gehalten. Alle drei Polyisocyanatharnstoff-Suspensionen haben nach dieser Zeit einen Gesamt-NCO-Gehalt von 6,1-6,2%. (ber. 6,2%). Aus der Höhe der Viskosität geht jedoch deutlich hervor, dass mit zunehmendem Anteil von 2,6-Diisocyanatotoluol die selektive Reaktion von Wasser erheblich gestört wird. Eine anteilmässige Mitvernetzung des höhermolekularen NCO-Präpolymeren ist insbesondere im Falle c) nicht auszuschliessen.

| | |
|---|---|
| Viskosität der | a) = 15 000 mPa.s/25°C |
| Polyisocyanatoharn- | b) = 18 000 mPa.s/25°C |
| stoff-Suspension | c) = 80 000 mPa.s/25°C |

Beispiel 3

Aus 2000 g eines linearen Polypropylenglykoläthers vom Molgewicht 2000 (OH-Zahl 56) und 522 g (3,0 Mol) 2,4-Diisocyanatotolul wird in an sich bekannter Weise ein NCO-Präpolymer mit überschüssigem aromatischem Diisocyanat hergestellt. Der NCO-Gehalt beträgt 6,6% (NCO berech. 6,7%). Zu 2000 g dieses NCO-Präpolymeren wird bei 50-60°C in zunehmender Menge Wasser zugesetzt und nach Beendigung der $CO_2$-Entwicklung (ca. 6 Stunden) der NCO-Gehalt und der freie 2,4-Diisocyanatotoluol-Anteil gemessen. Wie aus der Tabelle ersichtlich, fallen nach Zugabe von ca. 80% der für das freie niedermolekulare Diisocyanat berechneten Menge Wasser (7,0 g) die Polyisocyanatoharnstoffe aus und man erhält bei der weiteren Erhöhung des Wasserzusatzes Polyisocyanatharnstoff-Suspensionen mit nur noch sehr geringen Monomeranteilen.

| Wasser (g) | $CO_2$(l) | NCO (gesamt) | freies Diisocyanat | Phase |
|---|---|---|---|---|
| 4,3 | 5,0 | 4,6 | 2,0 | homogen |
| 5,0 | 5,3 | 4,0 | 1,5 | homogen |
| 5,7 | 5,8 | 4,0 | 0,75 | ↓ |
| 6,2 | | 3,95 | 0,55 | zunehmend |
| 6,7 | 6,8 | 3,9 | 0,5 | disperse |
| 7,0 | 7,4 | 3,8 | 0,4 | Phase |

Beispiel 4

Einem NCO-Präpolymeren, das aus 1000 g eines linearen Polypropylenglykoläthers vom Mol-Gewicht 2000 (OH-Zahl 56) und 258 g 2,4-Diisocyanatotoluol hergestellt wurde, und einen NCO-Gehalt von 9,8% aufweist, werden 9,5 g Pinakonhexahydrat auf einmal zugesetzt (entsprechend 0,54 Mol $H_2O$ pro Mol freiem TDI). Man erhält nach 5-6 Stunden bei 60-70°C eine Polyisocyanatharnstoff-Suspension vom Gesamt-NCO-Gehalt = 5,5% und einer Viskosität von 14 500 mPa.s/25°C. Es wurde 11 l $CO_2$ entwickelt.

Beispiel 5

Einem NCO-Präpolymeren, das aus 1000 g eines linearen Polypropylenglykoläthers vom Molgewicht 2000 und 168 g 1,6-Diisocyanatohexan hergestellt wurde und einen NCO-Gehalt von 3,5% aufweist, werden die in der folgenden Tabelle angegebenen Mengen an 2,4-Diisocyanatotoluol und Wasser zugesetzt. Man erhält Polyisocyanatharnstoff-Suspensionen mit den anführten Eigenschaften.

| | A | B | C |
|---|---|---|---|
| 2,4-Diisocyanatotoluol<br>Wasser | 348 g (2,0 Mol)<br>18 g (1,0 Mol) | 261 g (1,5 Mol)<br>13,5 g (0,75 Mol) | 174 g (1,0 Mol)<br>9 g (0,5 Mol) |
| Temperatur | 60° | 60° | 60° |
| $CO_2$-Menge (1) | 20 | 16 | 11 |
| Gesamt NCO (%) | 8,7 | 7,5 | 6,5 |
| NCO des isolierten Polyisocyanatharnstoffes (%) | 19,5 | 13,5 | 11,8 |
| Viskosität mPa.s/20°C | Paste, streichfähig | ca. 45000 | 10000 |

Beispiel 6

1000 g des im Beispiel 5 genannten NCO-Präpolymeren werden mit 126 g (0,5 Mol) 2,4'-Diisocyanatodiphenyläther vermischt und anschliessend werden 4,5 g Wasser zugesetzt. Nach 3 Stunden bei 60° werden 5,5 l $CO_2$ entwickelt und man erhält eine Polyisocyanatharnstoff-Suspension mit einem NCO-Gehalt von 5,3%.

Beispiel 7

Einem NCO-Präpolymeren, das aus 2000 g eines linearen Polyesters aus Adipinsäure und Äthylenglykol (Molgewicht 2000, OH-Zahl = 56) und 696 g (4,0 Mol) 2,4-Diisocyanatotoluol erhalten wurde (NCO = 9,4%), werden innerhalb von 1/2 Stunde bei 60° 18,0 g (1,0 Mol) Wasser zugetropft. Nach ca. 5 Stunden haben sich 19 l $CO_2$ entwickelt und die Polyisocyanatharnstoff-Suspension besitzt einen NCO-Gehalt von 6,1%.

Beispiel 8

In einer Versuchsreihe wurden einem NCO-Präpolymeren aus dem im Beispiel 7 genannten Polyester und 2,4-Diisocyanatotoluol (NCO = 3,8%) zusätzlich verschiedene Mengen 2,4-Diisocyanatotoluol zugemischt. Die anschliessende Reaktion mit Wasser führt in den in folgender Tabelle angegebenen Polyisocyanatharnstoff-Suspensionen, die in einem zweiten Reaktionsschritt mit einem aromatischen Diamin versetzt wurden (Beispiel 12)

| | A | B | C | D |
|---|---|---|---|---|
| NCO-Präpolymer NCO=3,8% (G) | 950 | 900 | 850 | 800 |
| 2,4-Diisocyanatotoluol (g) | 50 | 100 | 150 | 200 |
| Wasser (g) | 2,8 | 5,6 | 8,4 | 11,2 |
| Polyisocyanat-harnstoff-Suspension (NCO-Gehalt) | 4,9 | 6,0 | 7,1 | 8,15 |

Beispiel 9

1000 g des im Beispiel 5 genannten NCO-Präpolymeren werden mit 374 g eines Gemisches aus 60% 2,4'- und 40% 4,4'-Diisocyanatodiphenylmethan verrührt. Bei 60° werden innerhalb 3/4 Stunden 13,5 g Wasser zugetropft und der Reaktionsansatz wird nach 4 Stunden bei dieser Temperatur gehalten. Nach Entwicklung von 18 l $CO_2$ erhält man die Polyisocyanatharnstoff-Suspension in Form einer noch streichfähigen Paste. Der NCO-Gehalt beträgt 6,8%.

Beispiel 10

870 g (5,0 Mol) 2,4-Diisocyanatotoluol werden mit 3000 g eines verzweigten Polypropylenglykoläthers vom Molgewicht 3000 (Startmolekül = Trimethylolpropan, OH-Zahl = 56) vermischt und bei 80°C bis zu einem NCO-Gehalt von 7,5% erhitzt. Danach werden bei 60° 18 g Wasser innerhalb 1/2 Stunde zugetropft. Nach ca 6 Stunden werden 20 l $CO_2$ entwickelt und man erhält die Polyisocyanatharnstoff-Suspension mit einem NCO-Gehalt von 4,8% und einer Viskosität von 15 000 mPa . s/25°C.

Beispiel 11

100 Gewichtsteile der im Beispiel 1b hergestellten Polyisocyanatharnstoff-Suspension mit einem NCO-Gehalt von 6,0% werden bei 60-80° im Vakuum entgast und mit 17,3 g 3,5-Diäthyl-2,4-diaminotoluol innerhalb von 30 Sek. verrührt. Je die Hälfte des Reaktionsansatzes wird dann in eine auf 60° bzw. 120° heisse Metallform gegossen. Die Giesszeit beträgt ca. 2 Min. Nach etwa 10 Min. kann der Giessling entfernt werden. Nach Temperung von jeweis 24 Stunden bei 60° und bei 120° wurden die mechanischen Eigenschaften der Elastomeren bestimmt.

Temperatur

| | 60°C | 120°C |
|---|---|---|
| Zugfestigkeit (DIN 53504) | 8,5 | 19,5 MPa |
| Bruchdehnung (DIN 53504) | 380 | 550% |
| Weiterreissfestigkeit (DIN 53515) | 20,0 | 33 KN/m |
| Shore Härte A (DIN 53505) | 79 | 83 |
| Elastizität (DIN 53512) | 55 | 56% |

Aus der Zunahme der Zugfestigkeit, Bruchdehnung und Weiterreissfestigkeit bei ähnlicher Härte ist erkennbar, dass der zweite Reaktionsschritt, d.h. die Reaktion des Polyisocyanatharnstoffes mit dem aromatischen Diamin erst nach Temperung bei 120° vollständig abgelaufen ist.

Beispiel 12

100 Gew.-Teile der im Beispiel 8 hergestellten Polyisocyanatharnstoff-Suspension werden jeweils bei 80-100° im Vakuum entgast und anschliessend mit der angegebenen Menge 2,5-Diamino-4-chlor-benboesäureisobutylester innerhalb von 30 Sek. verrührt. Das $NCO/NH_2$-Molverhältnis liegt in allen Fällen bei 1,1:1. Die Reaktionsansätze werden in eingewachste und auf 100° erwärmte Formen ausgegossen. Nach einer Giesszeit von 2-5 Minuten erhält man nach einer Temperzeit von etwa 10 Stunden bei 120-130° Formkörper mit untenstehenden Eigenschaften.

| | NCO-Prepolymer NCO=3,8% | A | B | C | D |
|---|---|---|---|---|---|
| 2,5-Diamino-4-chlor-benzoesäure-isobutylester (9) | 10,0 | 12,8 | 15,7 | 18,4 | 21,4 |
| Giesszeit (Minuten) | 5 | 4,5 | 4 | 3,5 | 2 |
| Verfestigungszeit (Minuten) | 15 | 12 | 10 | 7 | 5 |
| Shore Härte D (DIN 53505) | 40 | 47 | 56 | 66 | 68 |
| Zugfestigkeit (M Pa) | 41,0 | 39,0 | 35,5 | 32,0 | 30,0 |
| Weiterreissfestigkeit (KN/m) | 68 | 72 | 75 | 95 | 98 |
| Elastizität (%) | 38 | 38 | 38 | 42 | 42 |

Bei zunehmenden Anteilen von Polyisocyanatharnstoff werden bei nahezu gleichbleibender Elastizität Härte und Weiterreissfestigkeit dieser Elastomere deutlich erhöht.

Beispiel 13

100 Gew.-Teile der im Beispiel 4 genannten Polyisocyanatharnstoff-Suspension mit einem NCO-Gehalt von 5,5% werden nach dem Entgasen bei 80°C mit 11,0, 3,5-Diäthyl-2,4-diaminotoluol verrührt. Nach einer Giesszeit von 2 Min. und einer Verfestigungszeit von 20 Min. erhält man einen elastischen Formkörper, der weitere 24 Stunden bei 120°C ausgeheizt wird und danach folgende mechanische Werte aufweist.

| | | |
|---|---|---|
| Zugfestigkeit (MPa) | = | 20,4 |
| Bruchdehnung (%) | = | 380 |
| Weiterreisswiderstand (KN/m) | = | 33,5 |
| Shore Härte A | = | 84 |
| Elastizität (%) | = | 49 |

Beispiel 14

Eine Mischung aus 100 Gew.-Teilen der in Beispiel 7 genannten Polyisocyanatharnstoff-Suspension, 12 g Butandiol-(1,4) und 0,2 g Stearylamid wird bei 80° mit 35,8 g geschmolzenem 4,4'-Diisocyanatodiphenylmethan verrührt und der Reaktionsansatz danach in auf 80° erwärmte Formen gegossen. Der thermoplastische PU-Prüfkörper wird nach einer Lagerung von etwa 2 Wochen bei 200-220° aufgeschmolzen und die Schmelzviskosität bestimmt. Aus der deutlichen Zunahme der Schmelzviskosität in einem Zeitraum von 30 Minuten geht hervor, dass nun die vollständige Reaktion des überschüssigen Butandiols-(1,4) mit dem heterogen vorliegenden Polyisocyanatharnstoff erfolgt und daher jetzt erst die mechanischen Endeigenschaften der Elastomeren erhalten werden.

**Patentansprüche**

1. Bei Raumtemperatur flüssige oder pastenförmige oder durch Erwärmen auf maximal 80°C verflüssigbare Suspensionen von

a) Isocyanatoharnstoffen der Formel

$$OCN\text{-}R\text{-}NH\text{-}CO\text{-}NH\text{-}[R\text{-}NH\text{-}CO\text{-}NH]_m\text{-}R\text{-}NCO$$

in welcher

R für einen zweiwertigen aromatischen Rest steht, wie er durch Entfernung der Isocyanatgruppen aus einem aromatischen Diisocyanat des Molekulargewichtsbereichs 174-400 mit einer sterisch ungehinderten aromatisch gebundenen Isocyanatgruppe und einer, durch mindestens einen Substituenten in o-Stellung sterisch gehinderten aromatisch gebundenen Isocyanatgruppe erhalten wird, wobei jedoch auch bis zu 50% der Reste R für einen solchen zweiwertigen aromatischen Rest stehen können, wie er durch Entfernung der Isocyanatgruppen aus einem aromatischen Diisocyanat mit Isocyanatgruppen gleicher Reaktivität erhalten wird und

m für 0 oder eine ganze Zahl von 1 bis 5 steht, in

b) Isocyanat-Präpolymeren der Formel

$$D\text{-}(O\text{-}CO\text{-}NH\text{-}A\text{-}NCO)_n$$

in welcher

A für einen gegebenenfalls Alkyl-substituierten aromatischen Kohlenwasserstoffrest mit insgesamt 6-15 Kohlenstoffatomen, einen aliphatischen Kohlenwasserstoffrest mit 4-10 Kohlenstoffatomen, einen cycloaliphatischen Kohlenwasserstoffrest mit 4-15 Kohlenstoffatomen oder einen Xylylenrest steht,

D für einen Ester- oder Äthergruppen aufweisenden Rest steht, wie er durch Entfernung der Hydroxylgruppen aus einem Polyester- oder Polyätherpolyol des Molekulargewichtsbereichs 500-8000 erhalten wird, und

n für eine ganze Zahl von 2 bis 4 steht.

2. Verfahren zur Herstellung von Suspensionen gemäss Anspruch 1, dadurch gekennzeichnet, dass man 5-40 Gew.-%, bezogen auf Gesamtgemisch, an aromatischen Diisocyanaten der Formel

$$R\ (NCO)_2$$

mit Isocyanatpräpolymeren der Formel

$$D\text{-}(O\text{-}CO\text{-}NH\text{-}A\text{-}NCO)_n$$

vermischt und anschliessend das Gemisch mit 0,4 bis 0,8 Mol Wasser pro Mol des aromatischen Diisocyanats oder einer entsprechenden Menge einer wasserabspaltenden Verbindung bei einer Temperatur zwischen 20 und 80°C zur Reaktion bringt, wobei A, D, R und n die in Anspruch 1 genannte Bedeutung haben.

3. Verwendung der Suspensionen gemäss Anspruch 1 als Isocyanatkomponente bei der Herstellung von Polyurethankunststoffen nach dem Isocyanat-Polyadditionsverfahren.

**Claims**

1. Suspensions which are liquid or paste-like at room temperature or which can be liquefied by heating to at most 80°C, of

a) isocyanate ureas of the formula

$$OCN\text{-}R\text{-}NH\text{-}CO\text{-}NH\text{-}[R\text{-}NH\text{-}CO\text{-}NH]_m\text{-}R\text{-}NCO$$

in which

R represents a difunctional aromatic radical of the type obtained by removing the isocyanate groups from an aromatic diisocyanate having a molecular weight in the range of from 174 to 400 and containing a sterically unhindered

aromatically bound isocyanate group and an aromatically bound isocyanate group which is sterically hindered by at least one substituent in the o-position, it also being possible for up to 50% of the radicals R to represent a difunctional aromatic radical of the type obtained by removing the isocyanate groups from an aromatic diisocyanate containing isocyanate groups of the same reactivity and

m represents 0 or an integer of from 1 to 5, in

b) isocyanate prepolymers of the formula

$$D\text{-}(O\text{-}CO\text{-}NH\text{-}A\text{-}NCO)\text{-}_n$$

in which

A represents an optionally alkyl-substituted aromatic hydrocarbon radical containing a total of 6-15 carbon atoms, an aliphatic hydrocarbon radical containing 4-10 carbon atoms, a cycloaliphatic hydrocarbon radical containing 4-15 carbon atoms or a xylylene radical,

D represents a radical containing ester or ether groups of the type obtained by removing the hydroxyl groups from a polyester or polyesther polyol having a molecular weight in the range of 500-8000, and

n represents an integer of from 2 to 4.

2. Process for producing the suspensions according to Claim 1, characterised in that 5-40% by weight, based on the total mixture, of aromatic diisocyanates of the formula

$$R\ (NCO)_2$$

are mixed with isocyanate prepolymers of the formula

$$D\text{-}(O\text{-}CO\text{-}NH\text{-}A\text{-}NCO)_n$$

and then the mixture is reacted with 0.4 to 0.8 mol of water per mol of the aromatic diisocyanate or with a corresponding quantity of a compound splitting off water, at a temperature between 20 and 80°C, A, D, R and n having the meaning given in Claim 1.

3. Use of the suspensions according to Claim 1 as the isocyanate component in the production of polyurethane plastics according to the isocyanate polyaddition process.

**Revendications**

1. Suspensions liquides ou pâteuses à la température ambiante ou liquéfiables par chauffage à 80°C au maximum,

a) d'isocyanato-urées de formule:

$$OCN\text{-}R\text{-}NH\text{-}CO\text{-}NH\text{-}[R\text{-}NH\text{-}CO\text{-}NH]_m\text{-}R\text{-}NCO$$

dans laquelle

R est un reste aromatique divalent, tel qu'on l'obtient en éliminant les groupes isocyanate d'un diisocyanate aromatique de poids moléculaire compris dans la plage de 174 à 400 portant un groupe isocyanate sans encombrement stérique, en liaison aromatique, et un groupe isocyanate en liaison aromatique, stériquement encombré par au moins un substituant en position ortho, jusqu'à 50% des restes R pouvant toutefois représenter un reste aromatique divalent tel qu'on l'obtient par élimination des groupes isocyanate d'un diisocyanate aromatique porteur de groupes isocyanate de même réactivité et

m est égal à 0 ou à un nombre entier de 1 à 5, dans

b) des prépolymères à groupes isocyanate de formule:

$$D\text{-}(O\text{-}CO\text{-}NH\text{-}A\text{-}NCO)\text{-}_n$$

dans laquelle

A désigne un reste d'hydrocarbure aromatique éventuellement porteur d'un substituant alkyle, totalisant 6 à 15 atomes de carbone, un reste d'hydrocarbure aliphatique ayant 4 à 10 atomes de carbone, un reste d'hydrocarbure cycloaliphatique ayant 4 à 15 atomes de carbone ou un reste xylylène,

D est un reste porteur de groupes ester ou éther, tel qu'on l'obtient par élimination des groupes hydroxyle d'un polyester- ou polyétherpolyol de poids moléculaire compris dans la plage de 500 à 8000, et

n est un nombre entier de 2 à 4.

2. Procédé de production de suspensions suivant la revendication 1, caractérisé en ce qu'on mélange 5 à 40% en poids, par rapport au mélange total, de diisocyanates aromatiques de formule:

$$R\ (NCO)_2$$

avec des prépolymères à groupes isocyanate de formule:

$$D\text{-}(O\text{-}CO\text{-}NH\text{-}A\text{-}NCO)_n$$

puis on fait réagir le mélange avec 0,4 à 0,8 mole d'eau par mole du diisocyanate aromatique ou avec une quantité correspondante d'un composé libérant de l'eau à une température comprise entre 20 et 80°C, A, D, R et n ayant la définition donnée dans la revendication 1.

3. Utilisation des suspensions suivant la revendication 1 comme composant isocyanate dans la production de matières plastiques du type polyuréthanne par le procédé de polyaddition d'un isocyanate.